# EUROPÄISCHE PATENTANMELDUNG

(11) **EP 1 006 111 A2**
(43) Veröffentlichungstag der Anmeldung: **07.06.2000**
(21) Anmeldenummer: 99123642.3
(22) Anmeldetag: 27.11.1999
(51) Int. Cl.: C07D 301/12, C07D 301/14, C07D 301/16

(54) **Verfahren zur enantioselektiven Epoxidierung von C=C-Doppelbindungen**

(30) Priorität: 03.12.1998 DE 19855858
(71) Anmelder: Degussa-Hüls Aktiengesellschaft, 60287 Frankfurt am Main (DE)
(72) Erfinder: Drauz, Karlheinz, Prof. Dr., 63579 Freigericht (DE); Roberts, Stan M., Prof. Dr., Parlegate, Neson 646RN (GB); Skidmore, John, Dr., Liverpool L73QB (GB)

(57) **Zusammenfassung**

Die vorliegende Erfindung richtet sich auf ein Verfahren zur enantioselektiven Epoxidierung von Verbindungen der allgemeinen Formel I 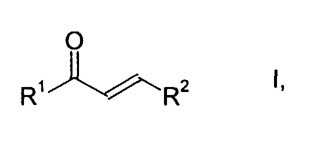 mittels einer diastereomeren- und enantiomerenangereicherten Homopolyaminosäure und einem Oxidans.

Verwendung der erfindungsgemäß hergestellten Epoxide als Intermediate in der organischen Synthese.

## Beschreibung

Die vorliegende Erfindung richtet sich auf ein Verfahren zur enantioselektiven Epoxidierung von C=C-Doppelbindungen. Insbesondere betrifft das Verfahren die Epoxidierung von Verbindungen der allgemeinen Formel I 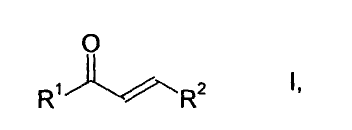 worin
R¹ und R² unabhängig voneinander bedeuten (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
   wobei die oben genannten Reste einfach oder mehrfach mit Heteroatomen wie Hal, NR³R⁴, PO₀₋₃R³R⁴, OR³, SR³, SOR³, SO₂R³, SO₃R³ substituiert sein können oder Gruppen wie CO₂R³, CONHR³ bzw. eine oder mehrere CH₂-Gruppen durch Heteroatome wie NR³, PR³, O oder S substituiert sein können,
R³ und R⁴ unabhängig voneinander bedeuten H, (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl,
   wobei die oben genannten Reste einfach oder mehrfach mit Hal substituiert sein können,
mittels einer diastereomeren- und enantiomerenangereicherten Homopolyaminosäure und einem Oxidans.

Enantioselektive Epoxidierungsreaktionen sind wichtige Reaktionen zum Aufbau chiraler Intermediate für die organische Synthese. Insbesondere die asymmetrische Epoxidierung von Allylalkoholen nach Sharpless et al. und die mangansalenvermittelte enantioselektive Epoxidierung nach Jacobsen et al. sind in der organischen Synthese zum Aufbau chiraler Moleküle wohl etabliert (Sharpless et al., J. Am. Chem. Soc. 1980, 102, 5974; J. Am. Chem. Soc. 1987, 109, 5765; J. Org. Chem. 1986, 51, 1922; Jacobsen et al., J. Am. Chem. Soc. 1990, 112, 2801; J. Am. Chem. Soc. 1991, 113, 7063).

Eine andere Möglichkeit zur asymmetrischen Epoxidation von C=C-Doppelbindungen wurde bei der Reaktion von Chalconen mit Wasserstoffperoxid in Gegenwart von enantiomerenangereicherten Polyaminosäuren entdeckt (Colonna et al., Org. Synth.; Mod. Trends, Proc. IUPAC Symp. 6th, 1986, 275; Julia et al., Angew. Chem., Int. Ed. Engel, 1980, 19, 929).

Die eben vorgestellten Synthesemethoden haben allesamt den Nachteil, daß sie auf ein relativ eng begrenztes Substratspektrum anwendbar sind. Aus dieser Tatsache und der fortwährenden Forschungstätigkeit auf diesem Gebiet ist die Notwendigkeit abzuleiten, weiterhin verbesserte Epoxidierungsprocedere zu finden.

Im Stande der Technik sind bis dato zwei unterschiedliche Varianten der Julia-Colonna-Epoxidierungsreaktion - die zweiphasige bzw. dreiphasige Variante - bekannt (S. M. Roberts et al. Chem. Commun. 1998, 1159; WO 96/33183). Die zweiphasige Variante bedient sich eines organischen Lösungsmittels und arbeitet mit in diesen Lösungsmitteln löslichen Oxidantien in Gegenwart der unlöslichen Homopolyaminosäuren. Die dreiphasige Variante benutzt neben dem wasserunlöslichen organischen Lösungsmittel ebenso Wasser als 3. Phase. So können vorteilhafterweise wasserlösliche Oxidantien für die Reaktion - ggf. in Gegenwart von Phasentransferkatalysatoren - herangezogen werden.

Aus den die letztgenannte Epoxidierungsreaktion betreffenden Schriften wird jedoch deutlich, daß entscheidende Mängel dieser Epoxidierungsmethoden im Hinblick auf deren Anwendung in einem industriellen Prozeß die zum Teil geringen Raum/Zeit-Ausbeuten (Reaktionszeiten im Bereich von Tagen) und die mitunter für manche Substrate schlechten ee-Werte sind.

Mithin war es Aufgabe der vorliegenden Erfindung, eine Möglichkeit zur Oxidation von C=C-Doppelbindungen aufzuzeigen, welche im Hinblick auf die chirale Induktionswirkung den Methoden des Standes der Technik nicht unterlegen ist, jedoch hinsichtlich der Raum/Zeit-Ausbeute, Handhabbarkeit und Ökonomie der Methode im technischen Maßstab Vorteile bietet.

Diese und nicht näher genannte weitere Aufgaben, die sich jedoch ohne weiteres und in naheliegender Weise aus dem Stand der Technik ergeben, werden durch ein Verfahren gelöst, dessen Merkmal Gegenstand des kennzeichnenden Teils des Anspruchs 1 ist. Bevorzugte Ausgestaltungen des erfindungsgemäßen Verfahrens sind in den von Anspruch 1 abhängigen Ansprüchen 2 bis 12 unter Schutz gestellt. Anspruch 13 bezieht sich auf eine erfindungsgemäße Verwendung.

Dadurch, daß die enantioselektive Epoxidierung von Verbindungen der allgemeinen Formel I 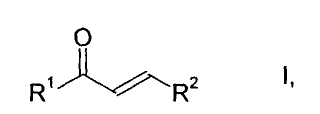 worin
R¹ und R² unabhängig voneinander bedeuten (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
   wobei die oben genannten Reste einfach oder mehrfach mit Heteroatomen wie Hal, NR³R⁴, PO₀₋₃R³R⁴, OR³, SR³, SOR³, SO₂R³, SO₃R³ substituiert sein können oder Gruppen wie CO₂R³, CONHR³ bzw. eine oder mehrere CH₂-Gruppen durch Heteroatome wie NR³, PR³, O oder S substituiert sein können,
R³ und R⁴ unabhängig voneinander bedeuten H, (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈) -Cycloalkyl, (C₁-C₈)-Alkyl-(C₃-C₈)-Cycloalkyl,
   wobei die oben genannten Reste einfach oder mehrfach mit Hal substituiert sein können,
mittels einer diastereomeren- und enantiomerenangereicherten Homopolyaminosäure und einem Oxidans dergestalt durchgeführt wird, daß die Epoxidierung in Gegenwart von Wasser und einem oder mehreren mit Wasser mischbaren organischen Lösungsmittel stattfindet, gelangt man in nicht ohne weiteres vorhersehbarer Weise und in einem vergleichsweise einfachen Prozeß in kürzeren Reaktionszeiten und mit hoher Ausbeute zu den gewünschten hoch enantiomerenangereicherten epoxidierten Derivaten. Gleichzeitig erlaubt die erfindungsgemäße Methode, Verbindungen einzusetzen, deren Grundgerüst sich nicht ausschließlich auf das der im Stand der Technik eingesetzten Chalcone reduziert.

Als wassermischbare organische Lösungsmittel werden bei dieser Reaktion vorzugsweise DMF, Acrylnitril, DMSO, wasserlösliche Alkohole oder wasserlösliche Ether eingesetzt. Besonders bevorzugt ist der Einsatz von DME. Die Lösungsmittel können alleine oder als Gemisch eingesetzt werden.

Das bei dieser Methode als Lösungsmittel eingesetzte einphasige Lösungsmittelgemisch ist somit verantwortlich für die vorteilhaften Eigenschaften dieser Reaktionsvariante. Mithin ist dieses Lösungsmittelsystem nicht mit den klassischen 2-/3-Phasensystemen vergleichbar, wird doch ein organisches Lösungsmittel wie beim 3-Phasen-Prozeß eingesetzt, doch erhält man während der Reaktion nur ein zweiphasiges System.

Die Wahl des Verhältnisses von Wasser zum organischen Lösungsmittel zur Erzielung der positiven Eigenschaften ist dabei relativ unkritisch. Bevorzugt kann ein Verhältnis von 10 : 1 bis 1 : 10 eingesetzt werden. Besonders bevorzugt ist ein Verhältnis von 5 : 1 bis 1 : 8, äußerst bevorzugt von 1 : 1 bis 1 : 5.

Als Substrate der allgemeinen Formel I können bevorzugt Verbindungen eingesetzt werden, in denen
- R¹: bedeutet (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, wobei die oben genannten Reste einfach oder mehrfach mit Hal oder NR³R⁴ substituiert sein können und
- R²: bedeutet (C₁-C₁₈) -Alkyl, (C₂-C₁₈) -Alkenyl, (C₆-C₁₈) -Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, wobei die oben genannten Reste einfach oder mehrfach mit Hal oder NR³R⁴ substituiert sein können sowie
- R³ und R⁴: die oben angegebene Bedeutung annehmen.

Zur Herstellung der enantiomerenangereicherten Epoxide können verschiedene diastereomeren- und enantiomerenangereicherten Homopolyaminosäuren eingesetzt werden. Vorzugsweise werden allerdings Homopolyaminosäuren aus der Gruppe Polyneopentylglycin, Polyleucin, Polyisoleucin, Polyvalin und Polyalanin sowie Polyphenylalanin benutzt. Aus dieser Gruppe ist Polyneopentylglycin am meisten zu bevorzugen.

Die Kettenlänge der Polyaminosäuren ist so zu wählen, daß einerseits die chirale Induktion bei der Reaktion nicht beeinträchtigt wird und andererseits die Kosten zur Synthese der Polyaminosäuren nicht zu groß werden. Vorzugsweise liegt die Kettenlänge der Homopolyaminosäuren zwischen 5 und 100, vorzugsweise 7 bis 50, Aminosäuren. Ganz besonders bevorzugt ist eine Kettenlänge von 10 bis 40 Aminosäuren.

Die Homopolyaminosäuren können unverändert in die Reaktion eingesetzt werden. Bevorzugt ist die Ausführungsform, in der die Homopolyaminosäuren mit polyfunktionellen Aminen vernetzt sind bzw. durch andere organische Polymere vergrößert sind. Vorteilhafterweise setzt man als vernetzende Amine 1,3-Diaminopropan, Propylenimintetraamindendrimere der 1. Generation oder quervernetztes Hydroxy- oder Aminopolystyrol (CLAMPS, kommerziell erhältlich) ein. Als Polymervergrößerer kommt bevorzugt polyethylenglykolpolystyrolbasierende Nucleophile in Frage. Derart veränderte Polyaminosäuren sind in Chem. Commun 1998, 1159f und Tetrahedron: Asymmetry 1997, 8, S. 3165f dargestellt.

Die unlöslichen Trägermaterialien sind solche, welche vorzugsweise auf Siliziumoxid-Basis aufgebaut sind, wie z. B. Molsieb, Silicagel oder Zeolithe sowie Celite 521® oder Celite Hyflo Super Cell®, Wessalith® Day P. Vorteilhaft sind auch Silicagele mit definierten Porengrößen wie z.B. CPC I oder CPC II. Bevorzugt ist auch Nitrocellulose oder Aktivkohle als Trägermaterial.

Das Verhältnis von Trägermaterial zu Polyaminosäure ist durch zwei Grenzen gegeben. Einerseits kann nur eine bestimmte Anzahl von Polyaminosäure auf dem unlöslichen Träger adsorbiert werden, auf der anderen Seite läßt die chirale Induktion ab weniger als 10 Gew.-% von Polyaminosäure zu Träger nach. Bevorzugt liegt das Verhältnis zwischen 1:7 und 2:1 Gew.-teilen, vorzugsweise bei 1:5 bis 1:1 Gew.-teilen.

Als Oxidantien, welche vorzugsweise im molaren Verhältnis zur zu oxidierenden Verbindung eingesetzt werden, bevorzugt ist ein Verhältnis von 1:1 bis 1:2, dienen in der Regel Peroxide, Persäuren, eine wäßrige Lösung von H₂O₂ oder anorganische Oxidationsmittel, wie Natriumperborat oder Natriumpercarbonat, vorzugsweise Na₂CO₃ · 1,5 H₂O₂. Weitere bei dieser Reaktion einzusetzende Oxidantien sind die in Houben-Weyl Band 4/1a+b, S. 59-319 sowie die in Oxidation in Organic Chemistry, Milos Hudlicky, ACS Monograph 186, Washington, DC 1990, S. 1-47 genannten Verbindungen.

Die Menge des bei dieser Reaktion einzusetzenden Katalysators kann in weiten Bereichen variiert werden. Sie wird im allgemeinen durch die Konzentration begrenzt, ab der die chirale Induktion beginnt nachzulassen bzw. ab der ein Einsatz des teuren Katalysators wirtschaftlich gesehen unsinnig wird. Im allgemeinen liegt das Molverhältnis von Katalysator zu Substrat zwischen einem Verhältnis von 1 : 1 bis 0,005 : 1. Besonders bevorzugt ist ein Katalysator-Verhältnis von 0,5 : 1 bis 0,05 : 1.

Die Temperatur, die bei der Epoxidierung zugegen ist, sollte zwischen -30 °C und 80 °C liegen. Vorzugsweise stellt man die Temperatur so ein, daß sie zwischen -10 °C und 50 °C, besonders bevorzugt zwischen 15 und 30 °C, liegt.

Der pH-Wert, welcher während der Reaktion eingestellt wird, kann so gewählt werden, daß ein Überschuß an deprotoniertem H₂O₂ verglichen mit nicht deprotoniertem H₂O₂ vorhanden ist. Andererseits sollte der pH-Wert auch bei der Reaktion nicht so hoch gewählt werden, daß die eingesetzten organischen Verbindungen Schaden nehmen. Vorzugsweise liegt der pH-Wert zwischen 7 und 14, bevorzugt zwischen 7,5 und 13.

Für die Erfindung vorteilhaft kann es sein, wenn dem Reaktionsgemisch aufweisend Substrat, Oxidans, Polyaminosäure und Lösungsmittelgemisch ein Phasentranferkatalysator zugesetzt wird. Als solche sind alle diejenigen Katalysatoren bevorzugt, die kationischer Natur sind, besonders bevorzugt sind quartäre Ammoniumverbindungen wie Aliquat 336, Triton B, etc. Weitere Katalysatoren sind in der Phase-Transfer Catalysis von C. M. Starks et al., Chapman&Hall London, 1990, S. 123-187 genannt.

Die nach Anspruch 1 hergestellten enantiomerenangereicherten Epoxide werden bevorzugt als chirale Intermediate in der organischen Synthese verwendet.

Die bei der Epoxidierung einzusetzenden Homopolyaminosäuren können nach Methoden des Standes der Technik hergestellt werden (Flisak et al., J. Org. Chem. 1993, 58, 6247). Die Methode ist auf beide optischen Antipoden der Aminosäuren anzuwenden. Der Einsatz einer bestimmten Antipode einer Polyaminosäure korreliert mit der Stereochemie des Epoxids, d. h. eine Poly-L-aminosäure führt zur optischen Antipode des Epoxids, das mit einer Poly-D-aminosäure erhalten wird.

Es hat sich gezeigt, daß eine Behandlung des polymeren Katalysator vor dessen Einsatz mit basisch wäßrigen Medien wie in der EP 0 403 252 A2 die Reaktionsgeschwindigkeit in der Epoxidierungsreaktion weiter absinken läßt.

Bei der vorliegenden erfindungsgemäßen Reaktion geht man im allgemeinen so vor, daß man die zu verwendende Homopolyaminosäure bzw. die abgeänderten Derivate, wie vernetzte, polymervergrößerte oder auf unlöslichen Trägern adsorbierte Homopolyaminosäure, in dem erfindungsgemäßen Lösungsmittelgemisch suspendiert, anschließend das Oxidans hinzufügt, den pH-Wert einstellt und abschließend das Substrat der allgemeinen Formel I zufügt. Die Einhaltung der Reihenfolge der Arbeitsschritte ist nicht zwingend, doch sollte das Substrat vorzugsweise als letztes zur Mischung hinzugefügt werden, um eine ungewollte nichtinduzierte Epoxidierung zu Beginn der Reaktion, wenn noch kein Katalysator beigemengt wurde, nicht zuzulassen, da dieses zu geringeren ee-Werten im Produkt Anlaß gibt.

Die Reaktionsgemische werden nach Verfahren, welche dem Fachmann bekannt sind, aufgearbeitet. Das lösliche Epoxid wird vorteilhafter Weise durch Filtration von der Polyaminosäure getrennt und anschließend wäßrig aufgearbeitet. Falls erwünscht, kann eine abschließende Chromatographie an Kieselgel zur Reinigung erfolgen.

Es hat sich gezeigt, daß der Katalysator recycliert und erneut in die Reaktion eingesetzt werden kann. In diesem Zusammenhang ist der Einsatz von polymervergrößerten oder vernetzten oder auf unlöslichen Trägern adsorbierten Polyaminosäuren besonders bevorzugt, da diese leichter handhab- und filtrierbar sind. Sie verstopfen nicht wie die freien Polyaminosäuren die Poren von Membranen und Filtern. Somit können derart modifizierte Katalysatoren äußerst vorteilhaft auch in einem Enzym-Membran-Reaktor (C. Wandrey in Enzymes as Catalysts in Organic Synthesis, Ed.: M. Schneider, Dordrecht Riedel 1986, 263-284) oder einem Rohrreaktor, wie z. B. einer Chromatographiesäule, eingesetzt werden, welche eine für einen industriellen Prozeß äußerst bevorzugte kontinuierliche bzw. quasikontinuierliche Fahrweise der Reaktion gestatten.

Für die Standardoperation - die Oxidation von Chalcon zu Epoxychalcon - sind die Ergebnisse der unterschiedlichen Methoden im folgenden mit Poly-L-leucin-CLAMPS (CLAMPS: cross-linked-amino-modified-polystyrene) als Katalysator gegenübergestellt (Schema 1).

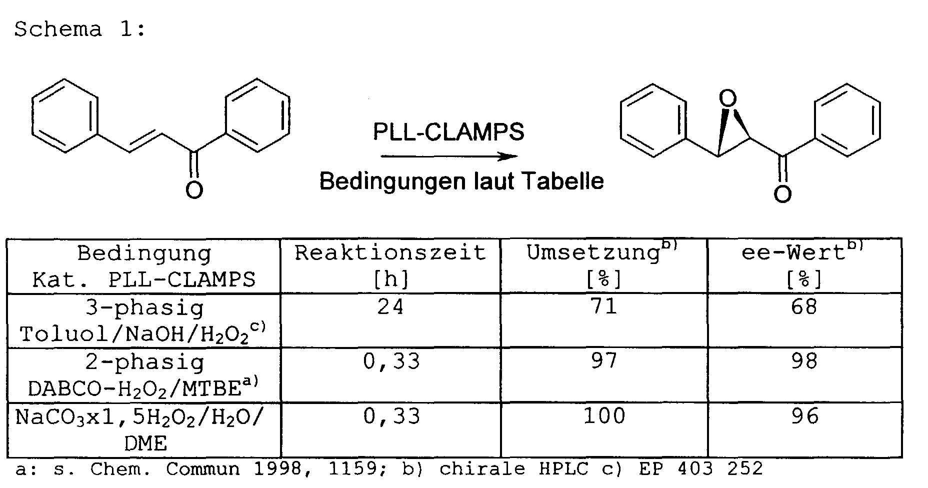

Wie aus den Ergebnissen ersichtlich ist, liegen die Reaktionszeiten der erfindungsgemäßen Methode weit unter denen, welche für die 3-phasige Variante angegeben sind. Die erfindungsgemäßen Ergebnisse und die der 2-phasigen Variante halten sich die Waage. Mithin ist aber der Einsatz der teuren Hilfsbase, welche für die zweiphasige Methode unverzichtbar ist (z. B. DABCO oder DBU), im großen Maßstab nachteilig. Zudem hat es sich gezeigt, daß beim up scaling der 2-phasigen Variante der ee-Wert mit steigender Substratmenge pro eingesetzter Katalysatormenge sinkt. Ein solches konnte bei der erfindungsgemäßen Methode nicht beobachtet werden (Schema 2).

| Schema 2: | | | | |
|---|---|---|---|---|
| Chalconmenge pro 100 mg Kat. | 2-phasig | | erfindungsgemäß | |
| | Ausbeute (%) | ee (%) | Ausbeute (%) | ee (%) |
| 50 mg | 98 | 97 | 99 | 95 |
| 300 mg | 92 | 88 | 87 | 95 |

Darüber hinaus ist festzustellen, daß mit der erfindungsgemäßen Methode auch geschützte Alkohole wie III 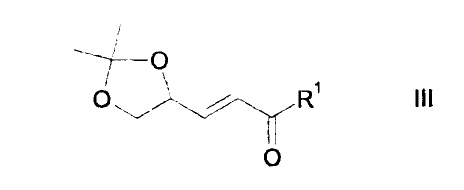 umzusetzen sind. Die Epoxidierung solcher Derivate war so aus dem Stand der Technik nicht bekannt und ist deshalb um so überraschender.

Durch die Verkürzung der Reaktionszeiten ist es jetzt nicht mehr nötig, die eingesetzten Oxidantien in irgendeiner Weise zu stabilisieren. Bei Reaktionszeiten des Standes der Technik, insbesondere der 3-phasigen Variante, die im Bereich von Tagen lagen, mußten häufig Chelatbildner zur Reaktionsmischung zugesetzt werden, da die Oxidantien sich in Gegenwart von Schwermetallionen allmählich zersetzten. Mitunter war auch die Nachdosierung von Oxidans notwendig, um die Reaktion einigermaßen zur Vollständigkeit zu bewegen. All dies braucht mit dem neuen Verfahren nicht beachtet zu werden. Dies ist mit Rücksicht auf dessen Anwendung im technischen Maßstab besonders vorteilhaft, spart man doch wichtige und teuere Einsatzstoffe ein.

Auch gegenüber der 2-phasigen Prozedur zeigt das erfindungsgemäße Verfahren wie oben dargelegt entscheidende Vorteile, insbesondere im Hinblick auf die Anwendung im industriellen Rahmen.

Die Erfindung ist deshalb mithin ursächlich für die Erhöhung der Attraktivität des Einsatzes dieser Reaktion im industriellen Maßstab, da die normalerweise langen Reaktionszeiten und die für die technische Größenordnung schlechten Ausbeuten, welche zur Kostenprogression der Produkte beitragen, auf akzeptable Werte zu bringen sind. Dies war äußerst überraschend und ist durch keinen Hinweis aus dem Stand der Technik nahegelegt.

Unter dem Begriff Homopolyaminosäuren versteht der Fachmann Polymere aus Aminosäuren einer Provenienz. Im Rahmen der Erfindung können die eingesetzten Homopolyaminosäuren jedoch auch Copolymere verschiedener Aminosäuren sein, bei der jedoch Domänen, welche die chirale Induktion bedingen, aus einheitlichen Aminosäuren bestehen sollten. Mithin bezieht sich der Ausdruck Homopolyaminosäuren auch auf aus heterochiralen Aminosäuren aufgebaute Polymere. Wiederum gilt, daß die Domänen, welche die chirale Induktion bedingen, aus einer stereochemisch einheitlichen Folge von Aminosäuren aufgebaut sein sollte.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. Insbesondere sind unter diese Gruppe die Reste Methyl, Ethyl, n-Propyl, Isopropyl, n-Butyl, Isobutyl, sec-Butyl, tert-Butyl, Pentyl, Hexyl etc. subsumierbar. Ein (C₁-C₆)-Alkylrest beschreibt den eben definierten Rest im Umfang von 1 bis 8 C-Atomen.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muß.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muß.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Rest angesehen, wie 1-, 2-, 3-Furyl, wie 1-, 2-, 3-Pyrrolyl, 1-,2-,3-Thienyl, 2-, 3-, 4-Pyridyl, 2-, 3-, 4-, 5-, 6-, 7-Indolyl, 3-, 4-, 5-Pyrazolyl, 2-,4-, 5-Imidazolyl, Acridinyl, Chinolinyl, Phenanthridinyl, 2-, 4-, 5-, 6-Pyrimidinyl, 4-, 5-, 6-, 7-(1-Aza)-indolizinyl.

Unter einem (C₄-C₁₉)-Heteroaralkyl wird ein dem (C₇-C₁₉)-Aralkylrest entsprechendes heteroaromtisches System verstanden.

Mithin bezeichnet im Rahmen der Erfindung ein (C₃-C₈)-Cycloalkylrest einen Rest der Gruppe der cyclischen Alkylreste mit 3 bis 8 C-Atomen und ggf. beliebiger Verzweigung. Insbesondere sind unter diese Gruppe die Reste Cyclopropyl, Cyclobutyl, Cyclopentyl, Cyclohexyl, Cycloheptyl zu subsumieren. In diesem Rest kann eine oder mehrere Doppelbindungen vorhanden sein.

Unter Hal versteht man Fluor, Chlor, Brom, Iod.

Unter dem Begriff enantiomerenangereichert wird im Rahmen der Erfindung der Anteil eines Enantiomers im Gemisch mit seiner optischen Antipode in einem Bereich von >50 % und <100 % verstanden.

Unter dem Begriff diastereomerenangereichert wird im Rahmen der Erfindung der Anteil eines Diastereomeren im Gemisch mit seinen anderen Diastereomeren in einem Bereich von >50 % und <100 % verstanden.

### Beispiele:

### Epoxidierung mit PLL

Zu einer gerührten Suspension von Chalcon (0.24 mmol) und PLL-CLAMPS (100 mg, Chem. Commun. 1998, 1159) in DME (0,5 cm³) und Wasser (0,5 cm³) werden Natriumpercarbonat (0,36 mmol) gegeben, und die Mischung wird für 20 min bei RT gerührt. Anschließend wird ein Aliquot entnommen und nach Filtration per HPLC (Chiralak AD-Säule, 10% EtOH in Hexan, 254nm, 1,0 ml/min Flußrate) analysiert.

Retentionszeiten der enantiomeren Epoxide: 15,9 (Hauptenantiomer) und 23,7

Ergebnisse siehe Schema 1.

## Patentansprüche

1. Verfahren zur enantioselektiven Epoxidierung von Verbindungen der allgemeinen Formel (I) 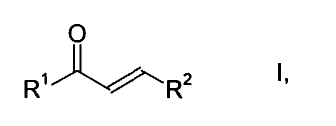 worin
R¹ und R² unabhängig voneinander bedeuten (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
wobei die oben genannten Reste einfach oder mehrfach mit Heteroatomen wie Hal, NR³R⁴, PO₀₋₃R³R⁴, OR³, SR³, SOR³, SO₂R³, SO₃R³ substituiert sein können oder Gruppen wie CO₂R³, CONHR³ bzw. eine oder mehrere CH₂-Gruppen durch Heteroatome wie NR³, PR³, O oder S substituiert sein können,
R³ und R⁴ unabhängig voneinander bedeuten H, (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl,
wobei die oben genannten Reste einfach oder mehrfach mit Hal substituiert sein können,
mittels einer diastereomeren- und enantiomerenangereicherten Homopolyaminosäure und einem Oxidans,
**dadurch gekennzeichnet,**
daß man die Epoxidierung in Gegenwart von Wasser und einem oder mehreren mit Wasser mischbaren organischen Lösungsmitteln durchführt.

2. Verfahren nach Anspruch 1,
**dadurch gekennzeichnet,**
daß man als organisches Lösungsmittel DMF, Acrylnitril, DMSO, wasserlösliche Alkohole oder wasserlösliche Ethern, vorzugsweise DME, verwendet.

3. Verfahren nach Anspruch 1 und/oder 2,
**dadurch gekennzeichnet,**
daß das Verhältnis von Wasser zu DME bei 10 : 1 bis 1 : 10, vorzugsweise 5 : 1 bis 1 : 8, liegt.

4. Verfahren nach einem oder mehreren der Ansprüche 1 bis 3,
**dadurch gekennzeichnet,**
daß man Verbindungen der allgemeinen Formel (I) worin
R¹ bedeutet (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl,
wobei die oben genannten Reste einfach oder mehrfach mit Hal oder NR³R⁴ substituiert sein können und
R² bedeutet (C₁-C₁₈)-Alkyl, (C₂-C₁₈)-Alkenyl, (C₆-C₁₈)-Aryl, (C₃-C₁₈)-Heteroaryl, (C₁-C₈)-Alkyl-(C₆-C₁₈)-Aryl, (C₁-C₈)-Alkyl-(C₃-C₁₉)-Heteroaryl, (C₃-C₈)-Cycloalkyl, (C₁-C₈)-Alkyl-(C₃-C₈)-Cycloalkyl,
wobei die oben genannten Reste einfach oder mehrfach mit Hal oder NR³R⁴ substituiert sein können und R³ und R⁴ die oben angegebene Bedeutung annehmen, umsetzt.

5. Verfahren nach einem oder mehreren der Ansprüche 1 bis 4,
**dadurch gekennzeichnet,**
daß man als Homopolyaminosäure eine Verbindung der Gruppe Polyneopentylglycin, Polyleucin, Polyisoleucin, Polyvalin, Polyphenylalanin und Polyalanin benutzt.

6. Verfahren nach einem oder mehreren der Ansprüche 1 bis 5,
**dadurch gekennzeichnet,**
daß die Homopolyaminosäure eine Kettenlänge von 5 bis 100 Aminosäuren, vorzugsweise 7 bis 50, besitzt.

7. Verfahren nach einem oder mehreren der Ansprüche 1 bis 6,
**dadurch gekennzeichnet,**
daß die Homopolyaminosäure bei der Epoxidierung frei, vernetzt, mit Polymeren vergrößert oder auf immobilisierten Trägern adsorbiert vorliegt.

8. Verfahren nach einem oder mehreren der Ansprüche 1 bis 7,
**dadurch gekennzeichnet,**
daß man als Oxidantien Peroxide, Persäuren, eine wäßrige Lösung von H₂O₂ oder anorganische Oxidantien, wie z.B. Natriumpercarbonat, Natriumperborat, benutzt.

9. Verfahren nach einem oder mehreren der Ansprüche 1 bis 8,
**dadurch gekennzeichnet,**
daß das Katalysatorverhältnis in einem Intervall von 1 : 1 bis 0,005 : 1, vorzugsweise 0,5 : 1 bis 0,05 : 1, bezogen auf das umzusetzende Substrat, liegt.

10. Verfahren nach einem oder mehreren der Ansprüche 1 bis 9,
**dadurch gekennzeichnet,**
daß die Temperatur bei der Epoxidierung zwischen 15°C und 30 °C liegt.

11. Verfahren nach einem oder mehreren der Ansprüche 1 bis 10,
**dadurch gekennzeichnet,**
daß man die Epoxidierung in einem pH-Intervall von 7 bis 14, vorzugsweise 7,5 bis 13, ausführt.

12. Verfahren nach einem oder mehreren der Ansprüche 1 bis 11,
**dadurch gekennzeichnet,**
daß man zum Epoxidierungsgemisch aufweisend Substrat, Oxidans, Polyaminosäure und Lösungsmittelgemisch ein Phasentranferkatalysator zusetzt.

13. Verwendung der gemäß einem oder mehrerer der Ansprüche 1 bis 12 hergestellten Epoxide als chirale Intermediate in der organischen Synthese.
